# EUROPÄISCHE PATENTANMELDUNG

(11) **EP 1 671 965 A2**
(43) Veröffentlichungstag der Anmeldung: **21.06.2006**
(21) Anmeldenummer: 06006969.7
(22) Anmeldetag: 03.04.2003
(51) Int. Cl.: C07D 321/12, A61K 31/357, A61P 3/00, A61P 19/00, A61P 21/00, A61P 25/14, A61P 25/16, A61P 25/28, A61P 35/00, A61P 43/00

(54) **Oxacarbocyclische Fumarsäure-Oligomere**

(30) Priorität: 18.04.2002 DE 10217314
(62) Teilanmeldung aus: 03712131.6
(71) Anmelder: Fumapharm AG, 6006 Luzern (CH)
(72) Erfinder: Joshi, Rajendra Kumar, Dr., 8047 Zürich (CH); Strebel, Hans-Peter, 5630 Muri (CH)
(74) Vertreter: Schwabe - Sandmair - Marx

(57) **Zusammenfassung**

Die vorliegende Erfindung betrifft bestimmte oxacarbocyclische Fumarsäure-Oligomere sowie die Verwendung derselben zur Herstellung eines Arzneimittels und diese Verbindungen enthaltende Arzneimittel.

## Beschreibung

Die vorliegende Erfindung betrifft bestimmte oxacarbocyclische Fumarsäure-Oligomere sowie die Verwendung derselben zur Herstellung eines Arzneimittels und diese Verbindungen enthaltende Arzneimittel.

Fumarsäuredialkylester sowie Fumarsäuremonoalkylester und Salze derselben werden seit langem mit Erfolg zur Behandlung der Psoriasis verwendet. Diese Verwendung ist in einer Anzahl von Patenten beschrieben worden, siehe beispielsweise die DE-25 30 372, DE 26 21 214 oder EP-B-0 312 697.

Weiterhin beschrieben ist die Verwendung der Fumarsäuremono- und -diester zur Behandlung von Autoimmunerkrankungen, wie beispielsweise der Polyarthritis oder der Multiplen Sklerose (siehe DE 197 21 099.6 sowie DE 198 53 487.6), aber auch zur Verwendung in der Transplantationsmedizin (siehe DE 198 53 487.6 und DE 198 39 566.3). Aus der DE 101 01 307.8 sowie der DE 100 00 577.2 ist außerdem die Verwendung der Fumarsäuremono- und -diester zur Behandlung NFkappaB vermittelter Erkrankungen wie die Behandlung mitochondrialer Erkrankungen bekannt. Alle genannten Druckschriften beschreiben Fumarsäuremono-und -diester, gegebenenfalls in Form bestimmter Salze.

Die genannten Fumarsäureester weisen wegen deren Flüchtigkeit bzw. Sublimierbarkeit den Nachteil auf, dass sie bei der Herstellung pharmazeutischer Produkte, insbesondere in fester Form zur oralen Verabreichung, schwierig zu handhaben sind. Genauer erfordert die Herstellung dieser Produkte Schutzmaßnahmen wie die Verwendung von Atemmasken, Handschuhen, Schutzanzügen usw.

Darüber hinaus werden die Fumarsäureester nach oraler Verabreichung im Magen-DarmTrakt resorbiert und von allen Körperzellen unspezifisch aus dem Blutstrom aufgenommen.

Es müssen daher entsprechend hohe Dosen verabreicht werden. Diese hohen Dosen führen wiederum zu den bekannten Nebenwirkungen der Fumarsäuretherapie wie Flush-Symptome (Rötungen) oder gastrointestinalen Reizungen (Übelkeit, Durchfälle, Blähungen). Die Nebenwirkungen können zwar, wie im oben genannten Stand der Technik beschrieben, durch die Verabreichung des Wirkstoffes in Form von Mikrotabletten erheblich gesenkt, jedoch nicht vollständig vermieden werden.

Gleichzeitig werden die Fumarsäureester im Blut rasch hydrolysiert und die Hydrolyseprodukte Alkohol und Fumarsäure bzw. Fumarsäuremonoester verstoffwechselt. Zum Erhalt therapeutisch wirksamer Spiegel ist daher eine wiederholte, häufig Verabreichung erforderlich. Zwar lässt sich bezüglich der Nebenwirkungen ein gewisser Gewöhnungseffekt beobachten, eine weitere Verringerung der Nebenwirkungsrate wäre jedoch wünschenswert.

Zur Vermeidung dieser Nachteile sind aus der nicht vorveröffentlichten DE 101 33 004.9 sind Fumarsäuremono- und -diamide bekannt. Diese Amide werden mit Aminosäuren und vorzugsweise mit bestimmten Peptiden gebildet. Die Bindung an ein Peptid soll für die spezifische Übermittlung des Fumarsäurederivats an einzelne Zielzellen sorgen. Die genannten Fumarsäure-Peptid-Derivate weisen jedoch den Nachteil auf, dass sie in der Herstellung sehr aufwendig sind.

Es ist daher Aufgabe der vorliegenden Erfindung, Fumarsäurederivate und deren Verwendung zur Verfügung zu stellen, die beständiger gegenüber der Hydrolyse und einfacher in Herstellung und Handhabung sind.

Gelöst wird die vorliegende Aufgabe durch bestimmte carbocyclische Fumarsäure-Oligomere, deren Verwendung zur Herstellung von Arzneimitteln und diese enthaltende Arzneimittel.

Genauer betrifft die vorliegende Erfindung in einem ersten Aspekt derselben daher carbocyclische Fumarsäure-Oligomere, enthaltend 2 bis 10 von Fumarsäure und/oder deren Estern und/oder Amiden abgeleitete Einheiten als wiederkehrende Einheiten. Diese carbocyclischen Fumarsäure-Oligomere werden vorzugsweise durch (olefinische) Polymerisation der C-C-Doppelbindungen der Einheiten erhalten. Vorzugsweise sind die von der Fumarsäure abgeleiteten Einheiten abgeleitet von Monomeren, ausgewählt aus der Gruppe, bestehend aus Fumarsäure, Dialkylfumaraten, Monoalkylhydrogenfumaraten, Fumarsäuremonoamiden, Fumarsäurediamiden, Monoalkylmonoamidofumaraten und deren Salzen sowie Mischungen derselben. Stärker bevorzugt enthält das erfindungsgemäße Oligomer lediglich von einem oder zwei Monomeren abgeleitete Einheiten, am meisten bevorzugt enthält das Oligomer ausschließlich identische Monomereinheiten. Bevorzugt handelt es sich bei dem Monomeren nicht um die Fumarsäure selbst, sondern um eines der genannten Derivate, insbesondere die Mono- oder Dialkylfumarate.

Das erfindungsgemäße carbocyclische Oligomer setzt sich aus den von der Fumarsäure abgeleiteten Einheiten dergestalt zusammen bzw. besteht aus diesen, dass die Einheiten an den C-Atomen 2 und 3 des Fumarsäureskeletts durch kovalente C-C-Bindungen so miteinander verbunden sind, dass ein carbocyclisches Oligomer entsteht. Die C-C-Bindungen können durch olefinische Polymerisation der Doppelbindungen entstehen. Das erfindungsgemäße carbocyclische Fumarsäure-Oligomer enthält vorzugsweise keine olefinischen Unsättigungen im Rückgrat.

Das Oligomerrückgrat (der Carbocyclus) besteht aus den Fumarsäure-Einheiten, d.h. es weist eine gerade Anzahl an C-Atomen auf und enthält keine anderen Monomeren und/oder Heteroatome. Dieses Rückgrat ist an jedem C-Atom durch eine der Carbonsäure- bzw. Carbonsäureamidgruppen der Fumarsäure-Monomereinheit(en) substituiert, aus der bzw. denen es aufgebaut ist. Die Monomereinheiten können bei der Synthese bzw. über Polymerisation der Derivate in Form der Ester oder Amide, aber auch in Form von Salzen vorliegen.

Der hier verwendete Ausdruck "Oligomer" bezieht sich auf eine Anzahl von mindestens zwei Fumarsäure-Monomereinheiten. Das erfindungsgemäße carbocyclische Fumarsäure-Oligomer enthält üblicherweise 2 bis 10, vorzugsweise 2 bis 6 und am meisten bevorzugt 2 bis 3 von Fumarsäure abgeleitete Einheiten. Diese können grundsätzlich in jeder sterischen Anordnung miteinander polymersieren bzw. zur Bildung des Carbocyclus aneinander gebunden sein. Bevorzugt stehen die Carbonsäure- bzw. Carbonsäureamidgruppen als Substituenten der Fumarsäure-Einheiten im erfindungsgemäßen Oligomer alle in trans-Stellung zueinander, d.h. zu beiden jeweils benachbarten Carbonsäure- bzw. Cabonsäureamidgruppen.

In einer bevorzugten Ausführungsform betrifft die Erfindung ein carbocyclisches Fumarsäure-Oligomer, entsprechend der folgenden Formel (I):

COR₁

-(-CH-CH-)n-

R₂OC

worin die Reste R1 und R2 gleich oder verschieden sind und ausgewählt sind unter Aminresten (-NR3R4), Aminosäureresten NH-C(COOH)-R6, Peptidresten mit 2 bis 100 Aminosäuren, Alkoholresten (-OR5) und einem Hydroxylrest,
n eine ganze Zahl von 2 bis 10 einschließlich ist,
die Reste R3 und R4 gleich oder verschieden sind und ausgewählt sind aus der Gruppe, bestehend aus Wasserstoff, C₁₋₂₄-Alkylresten, dem Phenylrest und C₆₋₁₀-Aralkylresten,
der Rest R5 ausgewählt ist aus der Gruppe, bestehend aus Wasserstoff, C₁₋₂₄-Alkylresten, dem Phenylrest und C₆₋₁₀-Aralkylresten, und
der Rest R6 eine Seitenkette einer natürlichen oder synthetischen Aminosäure darstellt.

In einer ersten Ausführungsform stellen die Reste R1 und R2 bevorzugt unabhängig je einen Alkohol- oder Hydroxylrest dar. Vorzugsweise bedeuten R1 und R2 nicht gleichzeitig Hydroxyl. Bei dem oder den Monomeren handelt es sich vorzugsweise also um ein oder mehrere Monoalkylhydrogenfumarat(e). In einer anderen Ausführungsform können beide Reste R1 und R2 einen Alkoxyrest -OR5 darstellen, der noch bevorzugter identisch ist. In diesem Fall handelt es sich bei dem oder den Monomeren um Dialkylfumarate.

Bevorzugt sind R1 und R2 unabhängig voneinander ausgewählt aus der Gruppe, bestehend aus Hydroxyl, Methoxy, Ethoxy, Propoxy, Isopropoxy, n-Butoxy, sec.-Butoxy, tert.-Butoxy, Phenoxy und Pentoxy, bevorzugt Methoxy und/oder Ethoxy. Ganz besonders bevorzugt sind demgemäß carbocyclische Oligomere die sich von Dimethylfumarat, Diethylfumarat, Methylethylfumarat, Methylhydrogenfumarat und Ethylhydrogenfumarat ableiten. Am meisten bevorzugt ist ein carbocyclisches Fumarsäure-Oligomer der Formel (I), worin R1 und R2 beide identisch Methoxy oder Ethoxy bedeuten.

Eine Carboxyfunktion tragende Monomere und die entsprechenden erfindungsgemäßen Polymere (in denen R1 und/oder R2 = -OH bzw. -O⁻ bedeuten) können selbstverständlich in Form ihrer Salze vorliegen. Bevorzugt sind die Alkalimetallsalze wie Li, Na, K, die Erdalkalimetallsalze wie Mg, Ca sowie die Salze physiologisch annehmbarer Übergangsmetalle, insbesondere Fe und Zn.

Nach einer dritten bevorzugten Ausführungsform betrifft die Erfindung ein carbocyclisches Oligomer, insbesondere der obigen Formel (I), worin R1 einen Aminrest -NR3R4 oder einen über eine Amidbindung gebundenen Aminosäurerest -NH-C(COOH)-R6 darstellt und R2 einen Aminrest -NR3R4, einen Alkoholrest -OR5 oder -OH darstellt.

Wie oben definiert kann der Rest R6 eine Seitenkette jeder natürlichen oder synthetischen Aminosäure darstellen. Der Aminosäurerest kann in der L- oder D-Konfiguration vorliegen, wobei die L-Konfiguration bevorzugt ist. Bevorzugt ist R6 ausgewählt aus der Gruppe, bestehend aus den Seitenketten von Ala, Val, Leu, Ile, Pro, Trp, Phe, Met, Gly, Ser, Tyr, Thr, Cys, Asn, Gln, Asp, Glu, Lys, His, Arg, Orn, Hcy, Hse, Hyp und Sar.

Bevorzugt sind die Reste R3, R4 und R5 gleich oder verschieden und sind ausgewählt aus der Gruppe, bestehend aus Wasserstoff, Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl, Vinyl, Allyl, 2-Hydroxyethyl, 2- oder 3-Hydroxypropyl, 2,3-Dihydroxypropyl, 2-Methoxyethyl, Methoxymethyl und 2- oder 3-Methoxypropyl.

In einer ganz besonders bevorzugten Ausführungsform betrifft die Erfindung ein carbocyclisches Fumarsäure-Oligomer, entsprechend der Formel (II): 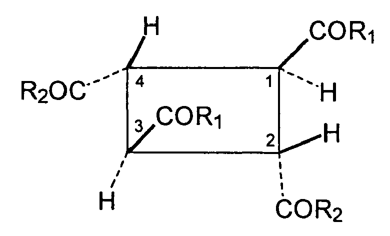 das als r-1,t-2,c-3,t-4-Tetrakis(alkoxycarbonyl)cyclobutan oder r-1,t-2,c-3,t-4-Cyclobutantetracarbonsäurealkylester bezeichnet werden kann,
oder entsprechend der Formel (III): 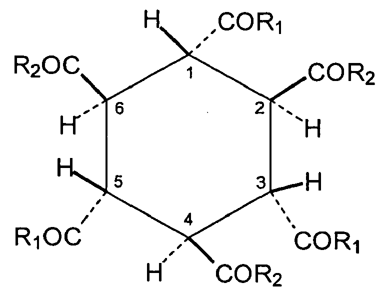 das als r-1,t-2,c-3,t-4,c-5,t-6-Hexa(alkoxycarbonyl)cyclohexan oder r-1,t-2,c-3,t-4,c-5,t-6-Cyclohexanhexacarbonsäurealkylester bezeichnet werden kann.

Am meisten bevorzugt betrifft die Erfindung ein carbocyclisches Fumarsäure-Oligomer, entsprechend der Formel (IIa): 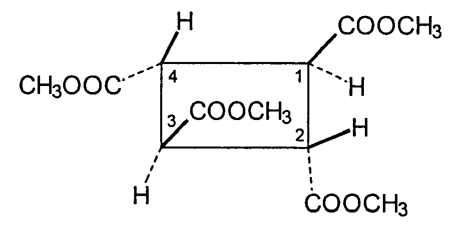 das als r-1,t-2,c-3,t-4-Tetrakis(methoxycarbonyl)cyclobutan oder r-1,t-2,c-3,t-4-Cyclobutan-tetracarbonsäuremethylester bezeichnet werden kann,
oder entsprechend der Formel (IIIa): 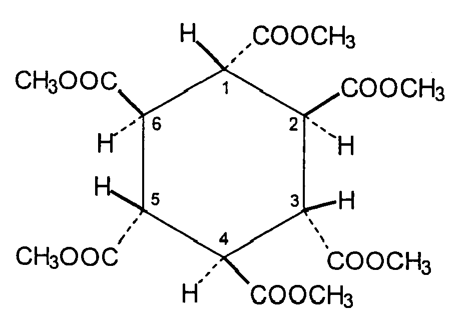 das als r-1,t-2,c-3,t-4,c-5,t-6-Hexa(methoxycarbonyl)cyclohexan oder r-1,t-2,c-3,t-4,c-5,t-6-Cyclohexanhexacarbonsäuremethylester bezeichnet werden kann.

Die erfindungsgemäßen carbocyclischen Fumarsäure-Oligomere können über allgemein bekannte Verfahren zur Herstellung zyklischer Verbindungen hergestellt werden. Die Herstellung kann beispielsweise mit Hilfe bekannter Zyklisierungsmittel wie Borverbindungen, Polyphosphorsäuren etc. unter üblichen Bedingungen erfolgen.

Bevorzugt werden die erfindungsgemäßen carbocyclischen Fumarsäure-Oligomere über ein Photopolymerisationsverfahren hergestellt. Wie bei diesen Verfahren üblich wird die Polymerisation durch Bestrahlung der Monomere, meist in flüssiger Phase ggf. in Kombination mit einem hierfür geeigneten, üblichen Lösungsmittel, das gegenüber der Polymerisation inert ist wie einem Alkan, Cycloalkan oder aromatischen Lösungsmittel, mit Licht einer Wellenlänge von 200 bis 700 nm induziert. Falls gewünscht können übliche Polymerisationsinitiatoren wie Hydroperoxide, organische Peroxide, Benzoinmethylether, Benzyl oder Diacetyl etc. und/oder Sensibilisierungsmittel zugesetzt werden, bspw. um die Ausbeute der Reaktion zu erhöhen. Zur Aktivierung der ethylenischen Unsättigungen der Fumarsäure-Monomereinheiten werden vorzugsweise Wellenlängen im UV- oder Blaulichtbereich verwendet.

Eine weitere bevorzugte Herstellungsmethode ist die sogenannte Metathese, die heute das Mittel der Wahl für gezielte Polymerisationen oder Ringschluss-Synthesen darstellt. Bei den als Metathesereaktionen bezeichneten Reaktionen handelt es sich im allgemeinen um von Schwermetallen katalysierte Zyklisierungen oder Polymerisationen. Einen allgemeinen Überblick gibt der Artikel "Die Olefinmetathese - neue Katalysatoren vergrößern das Anwendungspotential" von M. Schuster und S. Blechert, Chemie in unserer Zeit, Nr.1, 2001.

Die Metathesereaktionen zur Herstellung der erfindungsgemäßen Fumarsäure-Oligomere können unter Anwendung der üblichen Bedingungen und der üblichen Katalysatoren als homogene oder heterogene Reaktionen durchgeführt werden. Für die Katalysatoren sind beispielhaft die Katalysatoren auf Basis von Pd, Mo und Ru zu nennen, insbesondere der Grubb'sche Katalysator und der Schrock'sche Katalysator. Die Metathesen können in herkömmlichen Lösungsmitteln wie ggf. halogenierten Kohlenwasserstoffen, insbesondere Alkanen, Cycloalkanen, aromatischen Lösungsmitteln, aber auch Ethern, Estern, DMSO usw. durchgeführt werden. Die Reaktionstemperaturen liegen üblicherweise unterhalb von Raumtemperatur, beispielsweise zwischen -20 und 10 °C.

Die Herstellung der erfindungsgemäßen carbocyclischen Fumarsäure-Oligomere kann auch über Kombinationen der genannten Verfahren, beispielsweise zunächst über die Photopolymerisation zum Erhalt von cyclischen und/oder linearen Polymeren und anschließende Ringschlussmetathese, ggf. in Form der Abspaltung des zyklisierten Moleküls erfolgen (vgl. J. Pernerstorfer, M. Schuster und S. Blechert in "Cyclisation/cleavage of macrocycles by ring closing metathesis on solid support - confirmational studies", Chem. Commun. 1997, 1949).

In einem zweiten Aspekt betrifft die Erfindung die Verwendung eines wie oben definierten carbocyclischen Fumarsäure-Oligomeren zur Herstellung eines Arzneimittels. In einem dritten Aspekt betrifft die vorliegenden Erfindung außerdem ein Arzneimittel, enthaltend ein wie oben definiertes Fumarsäure-Oligomer.

Vorzugsweise ist das Arzneimittel zur Behandlung einer Autoimmunerkrankung, in der Transplantationsmedizin, zur Behandlung von mitochondrialen Erkrankungen und von NFkappaB beeinflußbaren Erkrankungen bestimmt. Das Arzneimittel ist vorzugsweise insbesondere bestimmt und geeignet
(1) zur Therapie einer Autoimmunerkrankung ausgewählt aus der Gruppe bestehend aus der Polyarthritis, Multiplen Sklerose, Graft-versus-Host-Reaktionen, dem juvenilen Diabetes, der Hashimoto-Tyreoiditis, der Grave's disease (Graves Krankheit oder Basedow Krankheit), dem systemischen Lupus erythematodes (SLE), dem Sjögren Syndrom (Sjogren's Syndrome), der perniziösen Anämie und der chronischen aktiven (= lupoiden) Hepatitis,
(2) zur Verwendung in der Transplantationsmedizin (Host-versus-Graft-Reaktionen),
(3) zur Therapie mitochondrialer Erkrankungen, ausgewählt aus der Gruppe bestehend aus dem Parkinson-Syndrom, der Alzheimer-Krankheit, der Chorea-Huntington-Krankheit, der Retinopathia pigmentosa oder mitochondrialen Enzephalomyopathien, sowie
(4) zur Therapie von NFkappaB vermittelten Erkrankungen, ausgewählt aus der Gruppe, bestehend aus der progressiven systemischen Sklerodermie, der Osteochondritis syphilitica (Wegener's Disease), der Cutis marmorata (Livedo Reticularis), der Behcet-Disease, Panarteritis, Colitis ulcerosa, Vasculitis, der Osteoarthritis, Gicht, Ateriosklerosis, der Reiter's Erkrankung, der bronchozentischen Granulomatose, Encephalitis-Typen, dem Endotoxin-Schock (septisch-toxischer Schock), der Sepsis, der Pneumonie, der Encephalomyelitis, der Anorexia nervosa, der Hepatitis (der akuten Hepatitis, der chronischen Hepatitis, der toxischen Hepatitis, der Alkoholhepatitis, der viralen Hepatitis, der Gelbsucht, der Leberinsuffizienz und der cytomegaloviralen Hepatitis), der Rennert T Lymphomatosis, der mesangialen Nephritis, der Postangioplastie-Restenose, das Reperfusionssyndrom, der cytomegaloviralen Retinopathie, Adenoviralen Erkrankungen wie adenoviralen Erkältungserkrankungen, dem adenoviralen Pharyngoconjunctivalfieber, und der adenoviralen Ophthalmie, AIDS, dem Guillain-Barre-Syndrom, der postherpetischen oder postzoster Neuralgie, der inflammatorischen demyelinisierenden Polyneuropathie, der Mononeuropathia multiplex, der Mukoviszidose, Morbus Bechterew, Barett-Ösophagus, EBV-(Epstein-Barr-Virus)-Infektion, dem kardialen Remodeling, interstitiellen Zystitis, Diabetes mellitus Typ II, der Strahlensensibilisierung maligner Tumore, der Mehrfachresistenz maligner Zellen auf Chemotherapeutika, Granuloma annulare und Krebserkrankungen wie Mamma Karzinom, Kolonkarzinom, Melanom, primäres Leberzellkarzinom, Adenokarzinom, Kaposi Sarkom, Prostatakarzinom, Leukämie wie der akuten myeloischen Leukämie, dem multiplen Myelom (Plasmozytom), Burkitt-Lymphom, und dem Castleman-Tumor.

Das Arzneimittel kann in für die orale, rektale, transdermale, ophtalmologische, nasale, pulmonale oder parenterale Applikation geeigneter Form vorliegen. Vorzugsweise ist das Arzneimittel für die orale Verabreichung geeignet. Es kann dann in Form von Tabletten, Dragees, Kapseln, Granulat, Trinklösungen, Liposomen, Nanopartikeln, Nanokapseln, Mikrokapseln, Mikrotabletten, Pellets oder Pulvern sowie in Kapseln gefülltem Granulat, in Kapseln gefüllten Mikrotabletten, in Kapseln gefüllten Pellets, in Kapseln gefüllten Nanopartikeln oder in Kapseln gefülltem Pulver vorliegen. Vorzugsweise liegt das Arzneimittel in Form von Nanopartikeln, Mikropellets oder Mikrotabletten vor, die ggf. in Sachets oder Kapseln abgefüllt sein können. Diese Mikropellets oder Mikrotabletten weisen üblicherweise einen Durchmesser, ohne Beschichtung, von≤ 5000 µm auf, vorzugsweise 300 bis 2000 µm.

Bevorzugt können alle festen oralen Dosisformen mit einer magensaftresistenten Beschichtung versehen sein. Diese kann beispielsweise auf den Tabletten, Mikrotabletten Mikropellets etc. aufgebracht sein, kann aber auch auf diese enthaltenen Kapseln aufgetragen werden.

Die erfindungsgemäßen Arzneimittelformen können grundsätzlich nach der klassischen Tablettiermethode aber auch über Direkttablettierung sowie feste Dispersionen nach der Schmelzmethode und über die Sprühtrocknungsmethode hergestellt werden. Ein magensaftresistenter Ueberzug kann, falls gewünscht, nach bekannten Verfahren in einem klassischen Dragierkessel aufgeleert oder aufgesprüht sowie in einer Wirbelschichtapparatur aufgetragen werden. Nach beendeter Trocknung kann anschliessend in der gleichen Apparatur ein Filmcoat aufgebracht werden.

Das erfindungsgemäße Arzneimittel enthält eine für den therapeutischen Zweck angemessene Menge an Oligomer. Diese kann von Fachmann anhand von Routineversuchen ermittelt werden. Üblicherweise wird das Arzneimittel eine Menge an Fumarsäure-Oligomer enthalten, entsprechend 10 bis 500 mg Fumarsäure, bevorzugt 30 bis 200 mg Fumarsäure und am meisten bevorzugt 100 mg Fumarsäure.

Die Verwendung der erfindungsgemäßen Oligomere bietet gegenüber der bekannten Verwendung der Monomere als Arzneimittelwirkstoffe den Vorteil, dass sie aufgrund des höheren Molekulargewichts weniger flüchtig sind und daher ein der Herstellung und Verarbeitung leichter zu handhaben sind. Sie bieten darüber hinaus den Vorteil, dass sie als synthetische Substanzen im Körper zunächst in körpereigene Substanzen überführt werden müssen, was ihre Verweilzeit in Organismus erhöht. Dies erfolgt vermutlich durch Aufspaltung in die Monomeren. Aufgrund der Oligomerisation weisen sie außerdem den Vorteil auf, dass sie weniger reizend auf die Schleimhäute wirken und damit weniger Nebenwirkungen aufweisen.

Die Erfindung soll nun anhand der folgenden Beispiele veranschaulicht werden, die diese jedoch nicht beschränken sollen.

### Beispiele

### Beispiel 1

### Herstellung von magensaftresistenten Mikrotabletten in Kapseln, enthaltend 60,0 mg r-1,t-2,c-3,t-4-Tetrakis(methoxycarbonyl)cyclobutan und 30.0 mg r-1,t-2,c-3,t-4,c-5, t-6 - Hexa(methoxycarbonyl)cyclohexan

6,0 kg r-1,t-2,c-3,t-4-Tetrakis(methoxycarbonyl)cyclobutan und 3,0 kg r-1,t-2,c-3,t-4, c-5, t-6 - Hexa(methoxycarbonyl)cyclohexan werden zerkleinert, intensiv gemischt und mittels eines Siebs 800 homogenisiert. Es wird ein Hilfsstoffgemisch folgender Zusammensetzung hergestellt: 18,00 kg Stärkederivat (STA-RX 1500), 0,30 kg Cellulose mikrokristallin (Avicel PH 101), 0,75 kg PVP (Kollidon 120), 4,00 kg Primogel, 0,25 kg Kieselsäure kolloidal (Aerosil). Das gesamte Pulvergemisch wird mit dem Wirkstoffgemisch versetzt und mittels eines Siebs 200 homogenisiert und mit einer 2%igen wässrigen Lösung von Polyvinylpyrrolidon (Kollidon K25) auf übliche Weise zu einem Bindemittelgranulat verarbeitet und in trockenem Zustand mit der äußeren Phase gemischt. diese besteht aus 0,50 kg Mg-Stearat und 1,50 kg Talkum. Das Pulvergemisch wird anschliessend auf üblicher Weise zu gewölbten Mikrotabletten von 10,0 mg Bruttomasse und 2,0 mm Durchmesser gepresst.

Anstelle dieser klassischen Tablettiermethode können auch andere Methoden zur Herstellung von Tabletten verwendet werden, wie Direkttablettierung sowie feste Dispersionen nach der Schmelzmethode und die Sprühtrocknungsmethode.

Der magensaftresistente Ueberzug kann in einem klassischen Dragierkessel aufgeleert oder aufgesprüht sowie in einer Wirbelschichtapparatur erfolgen. Zum Erreichen der Magensaftresistenz wird portionsweise eine Lösung von 2,250 kg Hydroxypropylmethylcellulosephthalat (HPMCCP, Pharmacoat HP 50), in einem Gemisch folgender Lösungsmittel auf gelöst: Aceton 13,001, Ethanol 94 Gewichtsprozent denaturiert mit 2% Keton 13,50 und Aqua demineralisata 2,50 1. Zu der fertigen Lösung wird als Weichmacher Rizinusöl 0,240 kg zugegeben und auf übliche Weise in Portionen auf die Tablettenkerne aufgetragen.

Filmcoat: Nach beendeter Trocknung wird anschliessend in der gleichen Apparatur eine Suspension folgender Zusammensetzung als Filmcoat aufgetragen: Talk 0,340 kg, Titan (VI)-oxyd Cronus RN 56 0,400 kg, Farblack L-Rotlack 86837 0,324 kg, Eudragit E 12,5% 4,800 kg und Polyethylenglycol 6000 pH 11 XI 0,120 kg in einem Lösungsgemisch folgender Zusammensetzung: 2-Propanol 8,170 kg, Aqua demineralisata 0,200 kg und Glycerintriacetat (Triacetin) 0,600 kg.

Die magensaftresistenten Mikrotabletten werden anschliessend in Hartgelatine-Steckkapseln zu 500,0 mg netto Gewicht eingefüllt und verschlossen.

### Beispiel 2

### Herstellung von magensaftresistenten Mikrotabletten in Kapseln, enthaltend 60,0 mg r-1,t-2,c-3,t-4-Tetrakis(methoxycarbonyl)cyclobutan und 30.0 mg r-1,t-2,c-3,t-4, c-5, t-6 - Hexa(methoxycarbonyl)cyclohexan

6,0 kg r-1,t-2,c-3,t-4-Tetrakis(methoxycarbonyl)cyclobutan und 3,0 kg r-1,t-2,c-3,t-4, c-5, t-6 - Hexa(methoxycarbonyl)cyclohexan werden zerkleinert, intensiv gemischt und mittels eines Siebs 800 homogenisiert. Es wird ein Hilfsstoffgemisch folgender Zusammensetzung hergestellt: 24,70 kg mikrokristalline Cellulose (Avicel PH 200), 3,00 kg Croscarmellose Natrium (AC-Di-SOL-SD-711), 2,50 kg Talkum, 0,10 kg Siliciumdioxid wasserfrei (Aerosil 200) und 1,00 kg Magnesiumstearat. Das gesamte Hilfsstoffgemisch wird mit dem Wirkstoffgemisch versetzt und homogen gemischt. Das Pulvergemisch wird anschliessend mittels Direkttablettierung zu gewölbten Mikrotabletten von 10,0 mg Bruttomasse und 2,0 mm Durchmesser gepresst.

Magensaftresistenter Ueberzug: Es wird eine Lösung von 0,94 kg Eudragit L in Isopropanol hergestellt, die zusätzlich 0,07 kg Dibutylphthalat enthält. Diese Lösung wird auf die Tablettenkerne aufgesprüht.

Danach wird eine Dispersion von 17,32 kg Eudragit L D-55 und einer Mischung aus 2,80 kg Mikrotalkum, 2,00 kg Macrogol 6000 und 0,07 kg Dimeticon in Wasser hergestellt und auf die Kerne aufgesprüht.

Die magensaftresistenten Mikrotabletten werden anschliessend in Hartgelatine-Steckkapseln oder in Beutel zu 760,0 mg netto Gewicht eingefüllt und verschlossen.

### Beispiel 3

### Herstellung von magensaftresistenten Pellets in Kapseln, enthaltend 60,0 mg r-1,t-2, c-3,t-4-Tetrakis(methoxycarbonyl)cyclobutan und 30.0 mg r-1,t-2,c-3,t-4, c-5, t-6 - Hexa(methoxycarbonyl)cyclohexan

6,0 kg r-1,t-2,c-3,t-4-Tetrakis(methoxycarbonyl)cyclobutan und 3,0 kg r-1,t-2,c-3,t-4, c-5, t-6 - Hexa(methoxycarbonyl)cyclohexan werden zerkleinert, intensiv gemischt und mittels eines Siebs 800 homogenisiert. Daneben wird 2 1 einer 20% (m/V) Polyvinylpyrrolidon (Kollidon K30) Lösung in Ethanol vorbereitet. 7,250 kg Nonpareilles Pellets werden in einem Dragierkessel belegt und mit einem Teil der Kollidon K-30 Lösung besprüht bis diese leicht feucht werden. Das Wirkstoffgemisch wird danach portionsweise zugegeben bis zum Auftrocknen der Pellets. Diese Vorgehensweise der Befeuchtung/Auftrocknung wird bis zur endgültigen Zugabe des Wirkstoffgemisches weitergeführt. Der Rest der PVP Lösung wird mit 0,720 kg Eudragit E 12.5% Lösung gemischt und ganz auf die Pellets gesprüht. Die Pellets werden bis zum vollständigen Austrocknen bewegt.

Die Pellets können auch mit den einzelnen Wirkstoffen hergestellt und nach Befilmung (siehe unten) in den entsprechenden Verhältnissen zugemischt werden.

Die Pellets werden mit Eudragit S 12.5% Lösung besprüht und mit Talk aufgetrocknet. Nach jedem Besprühungs-/Auftrocknungszyklus wird die Freisetzung des Wirkstoffes gemessen und Eudragit S 12.5% Lösung/Talkum weiter zugegeben bis eine Freisetzung gemäss Spezifikation erhalten wird.

Die magensaftresisteriten Pellets werden danach in Kapseln abgefüllt (150 mg Pellets pro Kapsel).

### Beispiel 4

### Herstellung von magensaftresistenten Tabletten, enthaltend 60,0 mg r-1,t-2,c-3,t-4-Tetrakis(methoxycarbonyl)cyclobutan und 30.0 mg r-1,t-2,c-3,t-4, c-5, t-6 - Hexa-(methoxycarbonyl)cyclohexan

6,0 kg r-1,t-2,c-3,t-4-Tetrakis(methoxycarbonyl)cyclobutan und 3,0 kg r-1,t-2,c-3,t-4, c-5, t-6 - Hexa(methoxycarbonyl)cyclohexan werden zerkleinert, intensiv gemischt und mittels eines Siebes 800 homogenisiert. Anschliessend wird ein Hilfsstoffgemisch folgender Zusammensetzung hergestellt: 20,000 kg Stärkederivat (STA-RX 1500® ), 2,000 kg mikrokristalline Cellulose (Avicel PH 101® ), 0,600 kg Polyvinylpyrolidon (PVP, Kollidon® 25), 4,000 kg Primogel® , 0,300 kg kolloidaler Kieselsäure (Aerosil® ).

Das gesamte Pulvergemisch wird mit dem Wirkstoff versetzt, gemischt, mittels eines Siebes 200 homogenisiert und mit einer 2%igen wässrigen Lösung von Polyvinylpyrolidon (PVP, Kollidon® 25) auf übliche Weise zu einem Bindemittelgranulat verarbeitet und in trockenem Zustand mit der äusseren Phase gemischt. Diese besteht aus 2,000 kg eines sogenannten FST-Komplexes, enthaltend 80% Talk, 10% Kieselsäure und 10% Magnesiumstearat. Es wird anschliessend auf übliche Weise zu gewölbten Tabletten von 500 mg Gewicht und 11,5 mm Durchmesser gepresst.

Es wird eine Lösung von 2,250 kg Hydroxypropylmethylcellulosephthalat (HPMCP, Pharmacoat HP® 50) in einem Lösungsmittelgemisch von 2,501 demineralisiertem Wasser, 13,00 Aceton Ph.Helv.VII und 13,00 Ethanol 94 Gewichtsprozent gelöst und die Lösung mit 0,240 kg Rizinusöl (Ph.Eur. II) versetzt. Die Lösung wird im Dragierkessel auf traditionelle Weise in Portionen auf die Tablettenkerne aufgeleert oder aufgesprüht.

Nach entsprechender Trocknung wird anschliessend der Filmüberzug angebracht. Dieser setzt sich zusammen aus einer Lösung von Eudragit E 12,5%® 4,800 kg, Talcum Ph. Eur. II 0,340 kg, Titan (VI)-oxyd Cronus RN 56® 0,520 kg, Farblack ZLT-2 blau (Siegle) 0,210 kg, und Polyethylenglycol 6000 Ph.Helv.VII 0,120 kg in einem Lösungsmittelgemisch von 8,200 kg 2-Propanol Ph.Helv.VII, 0,060 kg Glycerintriacetat (Triacetin® ) und 0,200 kg Aqua demineralisata. Nach homogener Verteilung im Dragierkessel oder Wirbelschichtbett wird getrocknet und auf übliche Weise poliert.

### Beispiel 5

### Herstellung einer Suspension zur parenteralen Applikation, enthaltend 60,0 mg r-1, t-2,c-3,t-4-Tetrakis(methoxycarbonyl)cyclobutan und 30.0 mg r-1,t-2,c-3,t-4, c-5,t-6 - Hexa(methoxycarbonyl)cyclohexan

| Inhaltsstoffe | mg/ml |
|---|---|
| r-1 ,t-2,c-3,t-4- Tetrakis(methoxycarbonyl)cyclobutan | 60.00 |
| r-1,t-2,c-3,t-4, c-5, t-6 - Hexa(methoxycarbonyl)cyclohexan | 30.00 |
| Methylcellulose | 0.25 |
| Natriumcitrat, Dihydrat | 30.00 |
| Benzylalkohol | 9.00 |
| Methylparaben | 1.80 |
| Propylparaben | 1.20 |
| Wasser für Injektionszwecke | q.s.a.d. 1.00 |

Unter Verwendung von Standard-Techniken werden die oben genannten Inhaltsstoffe zu einer parenteralen Suspension verarbeitet.

### Beispiel 6

### Herstellung einer Lösung zur parenteralen Applikation, enthaltend 30,0 mg r-1,t-2, c-3,t-4-Tetrakis (methoxycarbonyl)cyclobutan

| Inhaltsstoffe | Mg/ml |
|---|---|
| r-1,t-2,c-3,t-4-Tetrakis(methoxycarbonyl)cyclobutan | 30.00 |
| Hydroxypropyl-beta-cyclodextrin | 300.00 |
| Natriumdihydrogenphosphat | 10.00 |
| Methylparaben | 0.75 |
| Monothioglycerol | 2.00 |
| Wasser für Injektionszwecke | q.s.a.d. 1.00 |

Unter Verwendung von Standard-Techniken werden die oben genannten Inhaltsstoffe zu einer parenteralen Lösung verarbeitet.

Zusätzlich werden die folgenden Merkmalskombinationen (abgekürzt als MK) offenbart:
1. Carbocyclisches oder oxacarbocyclisches Oligomer, bestehend aus 2 bis 10 von Fumarsäure und/oder deren Estern und/oder Amiden abgeleiteten Einheiten als wiederkehrende Einheiten.
2. Carbocyclisches Oligomer nach MK 1, erhalten durch Polymerisation der C-C-Doppelbindungen der Einheiten.
3. Oxacarbocyclisches Fumarsäure-Oligomer nach MK 1, erhalten durch polarisierte olefinische Polymerisation der C-C-Doppelbindungen und Carbonylgruppen und enthaltend Etherbrücken im Carbocyclus.
4. Carbocyclisches Oligomer nach MK 1, 2 oder 3, worin die Fumarsäure-Einheiten abgeleitet sind von Monomeren, die ausgewählt sind aus der Gruppe, bestehend aus Fumarsäure, Dialkylfumaraten, Monoalkylhydrogenfumaraten, Fumarsäuremonoamiden, Fumarsäurediamiden, Monoalkylmonoamidofumaraten, deren Salzen sowie Mischungen derselben.
5. Carbocyclisches Fumarsäure-Oligomer nach MK 1 oder 2, entsprechend der folgenden Formel (I): 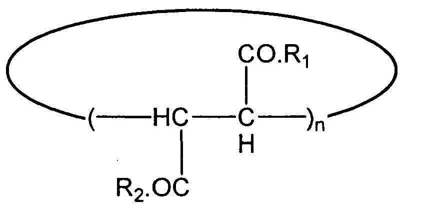 worin die Reste R1 und R2 gleich oder verschieden sind und ausgewählt sind aus der Gruppe, bestehend aus Aminresten (-NR3R4), Aminosäureresten (-NH-CH(COOH)-R6), Peptidresten mit 2 bis 100 Aminosäuren, Alkoholresten (-OR5) und einem Hydroxylrest,
   n eine ganze Zahl zwischen 2 und 10 ist,
   die Reste R3 und R4 gleich oder verschieden sind und ausgewählt sind aus der Gruppe, bestehend aus Wasserstoff, C₁₋₂₄-Alkylresten, dem Phenylrest und C₆₋₁₀-Aralkylresten,
   der Rest R5 ausgewählt ist aus der Gruppe, bestehend aus Wasserstoff, C₁₋₂₄₋Alkylresten, dem Phenylrest und C₆₋₁₀-Aralkylresten, und
   der Rest R6 die Seitenkette einer natürlichen oder synthetischen Aminosäure darstellt.
6. Oxacarbocyclisches Fumarsäure-Oligomer nach MK 1 oder 3, entsprechend der folgenden Formel (IV): 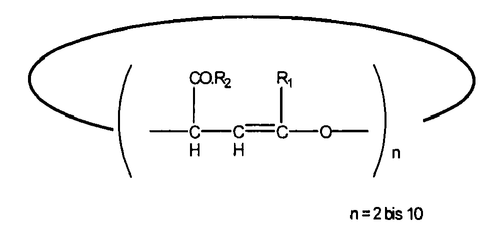 worin die Reste R1 und R2 gleich oder verschieden sind und ausgewählt sind aus der Gruppe, bestehend aus Aminresten (-NR3R4), Aminosäureresten (-NH-CH(COOH)-R6), Peptidresten mit 2 bis 100 Aminosäuren, Alkoholresten (-OR5) und einem Hydroxylrest,
   n eine ganze Zahl zwischen 2 und 10 ist,
   die Reste R3 und R4 gleich oder verschieden sind und ausgewählt sind aus der Gruppe, bestehend aus Wasserstoff, C₁₋₂₄-Alkylresten, dem Phenylrest und C₆₋₁₀-Aralkylresten,
   der Rest R5 ausgewählt ist aus der Gruppe, bestehend aus Wasserstoff, C₁₋₂₄₋Alkylresten, dem Phenylrest und C₆₋₁₀-Aralkylresten, und
   der Rest R6 die Seitenkette einer natürlichen oder synthetischen Aminosäure darstellt.
7. Oligomer nach MK 5 oder 6, worin die Reste R3, R4 und R5 gleich oder verschieden sind und ausgewählt sind aus der Gruppe, bestehend aus Wasserstoff, Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl, Vinyl, Allyl, 2-Hydroxyethyl, 2- oder 3-Hydroxypropyl, 2,3-Dihydroxypropyl, 2-Methoxyethyl, Methoxymethyl und 2- oder 3-Methoxypropyl.
8. Oligomer nach MK 5 oder 6, worin R1 einen Aminrest darstellt und R2 einen Alkoxyrest -OR5 oder -OH darstellt.
9. Oligomer nach MK 5 oder 6, worin R1 und R2 unabhängig je einen Alkoxy- oder Hydroxylrest darstellen.
10. Oligomer nach MK 9, worin R1 und R2 unabhängig voneinander ausgewählt sind aus der Gruppe, bestehend aus Methoxy, Ethoxy, Propoxy, Isopropoxy, n-Butoxy, sec.-Butoxy, tert.-Butoxy, Phenoxy und Pentoxy.
11. Oligomer nach MK 9, worin R1 und R2 beide Methoxy bedeuten.
12. Carboeyclisches oder Oxacarbocyclisches Oligomer nach einem der vorstehenden MK, enthaltend 2 bis 3 von Fumarsäure und/oder deren Estern und Amiden abgeleitete Einheiten.
13. Carbocyclisches Oligomer nach einem der vorstehenden MK, in dem alle die Reste R1 und R2 tragenden Carbonylgruppen als Substituenten in trans-Stellung zum jeweils benachbarten Substituenten angeordnet sind.
14. Carbocyclisches Oligomer nach MK 5, entsprechend der Formel (11): 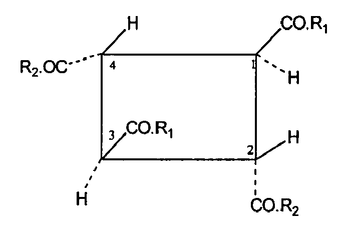 in der R1 und R2 wie in MK 5 definiert sind.
15. Carbocyclisches Oligomer nach MK 5, entsprechend der Formel (III): 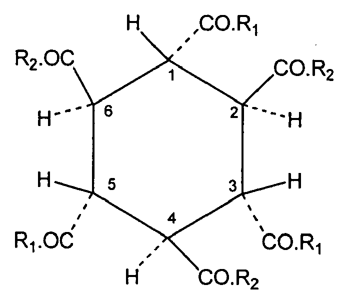 in der R1 und R2 wie in MK 5 definiert sind.
17. Oxacarbocyclisches Oligomer nach MK 6, entsprechend der Formel (IV): 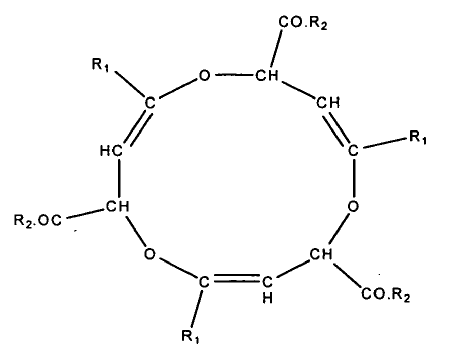 in der R1 und R2 wie in MK 6 definiert sind.
16. Oxacarbocyclisches Oligomer nach MK 6, entsprechend der Formel (V) 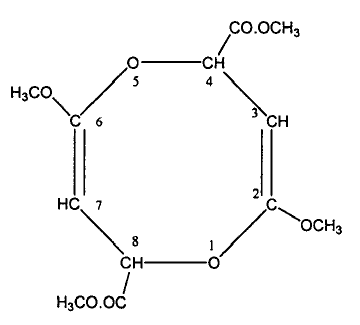
18. Verwendung eines carbocyclischen oder oxacarbocyclischen Oligomeren nach einem der vorstehenden MK zur Herstellung eines Arzneimittels.
19. Verwendung nach MK 18, worin das Arzneimittel bestimmt ist zur Behandlung einer Autoimmunerkrankung, in der Transplantationsmedizin, zur Behandlung von mitochondrialen Erkrankungen und von NFkappaB beeinflußbaren Erkrankungen.
20. Arzneimittel, enthaltend ein Fumarsäure-Oligomer nach einem der MK 1 bis 17.
21. Arzneimittel nach MK 20, wobei das Arzneimittel in für die orale, rektale, transdermale, dermale, ophtalmologische, nasale, pulmonale oder parenterale Applikation geeigneter Form vorliegt.
22. Arzneimittel nach MK 20, worin das Arzneimittel in Form von Tabletten, Dragees, Kapseln, Granulat, Trinklösungen, Liposomen, Nanopartikeln, Nanokapseln, Mikrokapseln, Mikrotabletten, Pellets oder Pulvern sowie in Kapseln gefülltem Granulat, in Kapseln gefüllten Mikrotabletten, in Kapseln gefüllten Pellets, in Kapseln gefüllten Nanopartikeln oder in Kapseln gefülltem Pulver vorliegt.
23. Arzneimittel nach MK 22, worin das Arzneimittel in Form von Nanopartikeln, Mikropellets oder Mikrotabletten vorliegt, die ggf. in Sachets oder Kapseln abgefüllt sein können.
24. Arzneimittel nach MK 22, worin die festen oralen Dosisformen mit einer magensaftresistenten Beschichtung versehen sind.
25. Arzneimittel nach einem der MK 20 bis 24, das eine Menge an Fumarsäure-Oligomer enthält, entsprechend 10 bis 500 mg Fumarsäure.
26. Verwendung nach einem der MK 19 bis 20, zur Herstellung eines Arzneimittels
   (4) zur Therapie einer Autoimmunerkrankung ausgewählt aus der Gruppe bestehend aus der Polyarthritis, Multiplen Sklerose, Graft-versus-Host-Reaktionen, dem juvenilen Diabetes, der Hashimoto-Tyreoiditis, der Grave's disease (Graves Krankheit oder Basedow Krankheit), dem systemischen Lupus erythematodes (SLE), dem Sjögren Syndrom (Sjogren's Syndrome), der perniziösen Anämie und der chronischen aktiven (= lupoiden) Hepatitis,
   (5) zur Verwendung in der Transplantationsmedizin,
   (6) zur Therapie mitochondrialer Erkrankungen, ausgewählt aus der Gruppe bestehend aus dem Parkinson-Syndrom, der Alzheimer-Krankheit, der Chorea-Huntington-Krankheit, der Retinopathia pigmentosa oder mitochondrialen Enzephalomyopathien, sowie
   (7) zur Therapie von NFkappaB vermittelten Erkrankungen, ausgewählt aus der Gruppe, bestehend aus der progressiven systemischen Sklerodermie, der Osteochondritis syphilitica (Wegener's Disease), der Cutis marmorata (Livedo Reticularis), der Behcet-Disease, Panarteritis, Colitis ulcerosa, Vasculitis, der Osteoarthritis, Gicht, Ateriosklerosis, der Reiter's Erkrankung, der bronchozentischen Granulomatose, Encephalitis-Typen, dem Endotoxin-Schock (septisch-toxischer Schock), der Sepsis, der Pneumonie, der Encephalomyelitis, der Anorexia nervosa, der Hepatitis (der akuten Hepatitis, der chronischen Hepatitis, der toxischen Hepatitis, der Alkoholhepatitis, der viralen Hepatitis, der Gelbsucht, der Leberinsuffizienz und der cytomegaloviralen Hepatitis), der Rennert T Lymphomatosis, der mesangialen Nephritis, der Postangioplastie-Restenose, das Reperfusionssyndrom, der cytomegaloviralen Retinopathie, Adenoviralen Erkrankungen wie adenoviralen Erkältungserkrankungen, dem adenoviralen Pharyngoconjunctivalfieber, und der adenoviralen Ophthalmie, AIDS, dem Guillain-Barre-Syndrom, der postherpetischen oder postzoster Neuralgie, der inflammatorischen demyelinisierenden Polyneuropathie, der Mononeuropathia multiplex, der Mukoviszidose, Morbus Bechterew, Barett-Ösophagus, EBV-(Epstein-Barr-Virus)-Infektion, dem kardialen Remodeling, interstitiellen Zystitis, Diabetes mellitus Typ II, der Strahlensensibilisierung maligner Tumore, der Mehrfachresistenz maligner Zellen auf Chemotherapeutika, Granuloma annulare und Krebserkrankungen wie Mamma Karzinom, Kolonkarzinom, Melanom, primäres Leberzellkarzinom, Adenokarzinom, Kaposi Sarkom, Prostatakarzinom, Leukämie wie der akuten myeloischen Leukämie, dem multiplen Myelom (Plasmozytom), Burkitt-Lymphom, und den Castleman-Tumor.

## Patentansprüche

1. Oxacarbocyclisches Oligomer, bestehend aus 2 bis 10 von Fumarsäure und/oder deren Estern und/oder Amiden abgeleiteten Einheiten als wiederkehrende Einheiten.

2. Oxacarbocyclisches Fumarsäure-Oligomer nach Anspruch 1, erhalten durch polarisierte olefinische Polymerisation der C-C-Doppelbindungen und Carbonylgruppen und enthaltend Etherbrücken im Carbocyclus.

3. Oxacarbocyclisches Fumarsäure-Oligomer nach Anspruch 1 oder 2, entsprechend der folgenden Formel (IV): 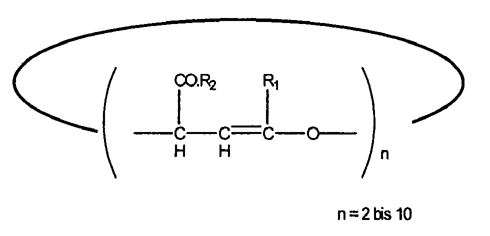 worin die Reste R1 und R2 gleich oder verschieden sind und ausgewählt sind aus der Gruppe, bestehend aus Aminresten (-NR3R4), Aminosäureresten (-NH-CH(COOH)-R6), Peptidresten mit 2 bis 100 Aminosäuren, Alkoholresten (-OR5) und einem Hydroxylrest,
n eine ganze Zahl zwischen 2 und 10 ist, .
die Reste R3 und R4 gleich oder verschieden sind und ausgewählt sind aus der Gruppe, bestehend aus Wasserstoff, C₁₋₂₄-Alkylresten, dem Phenylrest und C₆₋₁₀-Aralkylresten,
der Rest R5 ausgewählt ist aus der Gruppe, bestehend aus Wasserstoff, C₁₋₂₄₋Alkylresten, dem Phenylrest und C₆₋₁₀-Aralkylresten, und
der Rest R6 die Seitenkette einer natürlichen oder synthetischen Aminosäure darstellt.

4. Oligomer nach Anspruch 3, worin die Reste R3, R4 und R5 gleich oder verschieden sind und ausgewählt sind aus der Gruppe, bestehend aus Wasserstoff, Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl, Vinyl, Allyl, 2-Hydroxyethyl, 2- oder 3-Hydroxypropyl, 2,3-Dihydroxypropyl, 2-Methoxyethyl, Methoxymethyl und 2- oder 3-Methoxypropyl.

5. Oligomer nach Anspruch 4, worin R1 einen Aminrest darstellt und R2 einen Alkoxyrest -OR5 oder -OH darstellt.

6. Oligomer nach Anspruch 3, worin R1 und R2 unabhängig je einen Alkoxy- oder Hydroxylrest darstellen.

7. Oligomer nach Anspruch 6, worin R1 und R2 unabhängig voneinander ausgewählt sind aus der Gruppe, bestehend aus Methoxy, Ethoxy, Propoxy, Isopropoxy, n-Butoxy, sec.-Butoxy, tert.-Butoxy, Phenoxy und Pentoxy.

8. Oligomer nach Anspruch 6, worin R1 und R2 beide Methoxy bedeuten.

9. Oxacarbocyclisches Oligomer nach einem der vorstehenden Ansprüche, enthaltend 2 bis 3 von Fumarsäure und/oder deren Estern und Amiden abgeleitete Einheiten.

10. Oxacarbocyclisches Oligomer nach Anspruch 3, entsprechend der Formel (IV): 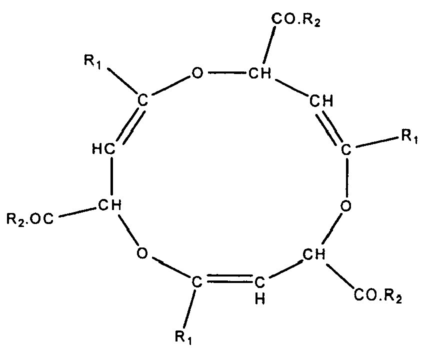 in der R1 und R2 wie in Anspruch 3 definiert sind.

11. Oxacarbocyclisches Oligomer nach Anspruch 3, entsprechend der Formel (V)

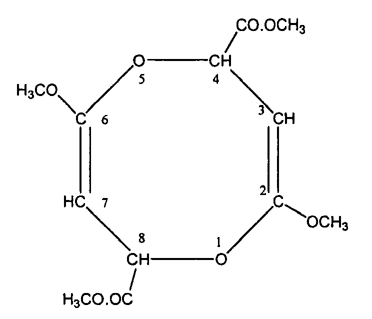

12. Verwendung eines oxacarbocyclischen Oligomeren nach einem der vorstehenden Ansprüche zur Herstellung eines Arzneimittels.

13. Verwendung nach Anspruch 12, worin das Arzneimittel bestimmt ist zur Behandlung einer Autoimmunerkrankung, in der Transplantationsmedizin, zur Behandlung von mitochondrialen Erkrankungen und von NFkappaB beeinflußbaren Erkrankungen.

14. Arzneimittel, enthaltend ein Fumarsäure-Oligomer nach einem der Ansprüche 1 bis 11.

15. Arzneimittel nach Anspruch 14, wobei das Arzneimittel in für die orale, rektale, transdermale, dermale, ophtalmologische, nasale, pulmonale oder parenterale Applikation geeigneter Form vorliegt.

16. Arzneimittel nach Anspruch 14, worin das Arzneimittel in Form von Tabletten, Dragees, Kapseln, Granulat, Trinklösungen, Liposomen, Nanopartikeln, Nanokapseln, Mikrokapseln, Mikrotabletten, Pellets oder Pulvern sowie in Kapseln gefülltem Granulat, in Kapseln gefüllten Mikrotabletten, in Kapseln gefüllten Pellets, in Kapseln gefüllten Nanopartikeln oder in Kapseln gefülltem Pulver vorliegt.

17. Arzneimittel nach Anspruch 16, worin das Arzneimittel in Form von Nanopartikeln, Mikropellets oder Mikrotabletten vorliegt, die ggf. in Sachets oder Kapseln abgefüllt sein können.

18. Arzneimittel nach Anspruch 16, worin die festen oralen Dosisformen mit einer magensaftresistenten Beschichtung versehen sind.

19. Arzneimittel nach einem der Ansprüche 14 bis 18, das eine Menge an Fumarsäure-Oligomer enthält, entsprechend 10 bis 500 mg Fumarsäure.

20. Verwendung nach einem der Ansprüche 13 oder 14, zur Herstellung eines Arzneimittels
(1) zur Therapie einer Autoimmunerkrankung ausgewählt aus der Gruppe bestehend aus der Polyarthritis, Multiplen Sklerose, Graft-versus-Host-Reaktionen, dem juvenilen Diabetes, der Hashimoto-Tyreoiditis, der Grave's disease (Graves Krankheit oder Basedow Krankheit), dem systemischen Lupus erythematodes (SLE), dem Sjögren Syndrom (Sjogren's Syndrome), der perniziösen Anämie und der chronischen aktiven (= lupoiden) Hepatitis,
(2) zur Verwendung in der Transplantationsmedizin,
(3) zur Therapie mitochondrialer Erkrankungen, ausgewählt aus der Gruppe bestehend aus dem Parkinson-Syndrom, der Alzheimer-Krankheit, der Chorea-Huntington-Krankheit, der Retinopathia pigmentosa oder mitochondrialen Enzephalomyopathien, sowie
(4) zur Therapie von NFkappaB vermittelten Erkrankungen, ausgewählt aus der Gruppe, bestehend aus der progressiven systemischen Sklerodermie, der Osteochondritis syphilitica (Wegener's Disease), der Cutis marmorata (Livedo Reticularis), der Behcet-Disease, Panarteritis, Colitis ulcerosa, Vasculitis, der Osteoarthritis, Gicht, Ateriosklerosis, der Reiter's Erkrankung, der bronchozentischen Granulomatose, Encephalitis-Typen, dem Endotoxin-Schock (septisch-toxischer Schock), der Sepsis, der Pneumonie, der Encephalomyelitis, der Anorexia nervosa, der Hepatitis (der akuten Hepatitis, der chronischen Hepatitis, der toxischen Hepatitis, der Alkoholhepatitis, der viralen Hepatitis, der Gelbsucht, der Leberinsuffizienz und der cytomegaloviralen Hepatitis), der Rennert T Lymphomatosis, der mesangialen cytomegaloviralen Retinopathie, Adenoviralen Erkrankungen wie adenoviralen Erkältungserkrankungen, dem adenoviralen Pharyngoconjunctivalfieber, und der adenoviralen Ophthalmie, AIDS, dem Guillain-Barré-Syndrom, der postherpetischen oder postzoster Neuralgie, der inflammatorischen demyelinisierenden Polyneuropathie, der Mononeuropathia multiplex, der Mukoviszidose, Morbus Bechterew, Barett-Ösophagus, EBV-(Epstein-Barr-Virus)-Infektion, dem kardialen Remodeling, interstitiellen Zystitis, Diabetes mellitus Typ II, der Strahlensensibilisierung maligner Tumore, der Mehrfachresistenz maligner Zellen auf Chemotherapeutika, Granuloma annulare und Krebserkrankungen wie Mamma Karzinom, Kolonkarzinom, Melanom, primäres Leberzellkarzinom, Adenokarzinom, Kaposi Sarkom, Prostatakarzinom, Leukämie wie der akuten myeloischen Leukämie, dem multiplen Myelom (Plasmozytom), Burkitt-Lymphom, und den Castleman-Tumor.
